# FASCICULE DE BREVET EUROPEEN

(11) **EP 3 185 821 B1**
(45) Date de publication et mention de la délivrance du brevet: **30.10.2019**
(21) Numéro de dépôt: 15775494.6
(22) Date de dépôt: 24.08.2015
(51) Int. Cl.: A61F 2/46

(54) **CORPS D'IMPACTEUR POUR OPERATION DE CHIRURGIE ORTHOPEDIQUE**
IMPAKTOR FÜR DIE ORTHOPÄDISCHE CHIRURGIE
IMPACTOR BODY FOR ORTHOPAEDIC SURGERY OPERATION

(30) Priorité: 26.08.2014 WO PCT/IB2014/064062
(43) Date de publication de la demande: 05.07.2017
(73) Titulaire: MPS Precimed SA, 2504 Biel/Bienne (CH)
(72) Inventeur: THULIEZ, Jean-Luc, 2525 Le Landeron (CH); OBLIGER, Nicolas, 2523 Lignières (CH); CROZIER, Etienne, 2520 La Neuveville (CH); ROSSE, Yann, 2520 La Neuveville (CH)
(74) Mandataire: Grosfillier, Philippe
(86) Numéro de dépôt international: PCT/IB2015/056408
(87) Numéro de publication internationale: WO 2016/030809

(56) Documents cités:
- WO-A1-2010/046634
- US-A1- 2009 192 515
- US-A1- 2013 226 186
- US-B1- 7 335 207

## Description

### Domaine technique

La présente invention concerne les opérations chirurgicales de la hanche et plus précisément les impacteurs utilisés à cet effet. L'état de la technique le plus proche est donné par le document US 2009/0192515 A1, qui définit le préambule de la revendication 1.

### Etat de la technique

Les impacteurs sont des outils orthopédiques utilisés pour la mise en place de cupules orthopédiques dans des logements préparés à cet effet dans l'os du bassin.

La cupule est initialement fixée sur une extrémité de l'impacteur. Le chirurgien utilise l'impacteur pour positionner la cupule en face du logement précédemment préparé. Il frappe ensuite sur l'extrémité opposée à la cupule pour forcer celle-ci à pénétrer au fond de son logement. Une fois la cupule en place, le chirurgien la désolidarise de l'impacteur et retire ce dernier, laissant la cupule dans son logement.

Les corps d'impacteur ne sont généralement pas linéaires. Ils présentent un décalage dans leur partie médiane afin de limiter la longueur de l'incision dans les tissus du patient. Ils doivent permettrent le montage des autres pièces constituant l'impacteur, telles que la chaîne cinématique permettant la fixation et le verrouillage de la cupule. Ils doivent être résistants mécaniquement pour permettre une bonne manipulation par le chirurgien. Ils doivent posséder une bonne résistance aux impacts pour supporter les coups de marteau. Ils doivent enfin être biocompatibles pour éviter toute contamination au patient.

Les corps d'impacteur de l'état de la technique sont réalisés en acier inoxydable, usinés pour la plupart. Les aciers utilisés présentent en effet une bonne résistance mécanique, une bonne résistance aux chocs, des propriétés de biocompatibilité suffisantes et une bonne tenue à la stérilisation, propriété nécessaire puisqu'ils ne sont pas à usage unique.

Un corps d'impacteur est coûteux à réaliser. Son procédé d'usinage n'offre pas beaucoup de liberté de design. En outre sa masse le rend peu maniable.

Enfin, le guidage de la chaîne cinématique est complexe, coûteux à fabriquer et complique fortement le nettoyage et la stérilisation des impacteurs existants.

### Exposé de l'invention

La présente invention est définie dans la revendication 1 et permet de pallier à l'ensemble des inconvénients mentionnés ci-dessus. En conséquence, un objectif de l'invention consiste à produire un corps d'impacteur de préférence biocompatible, résistant aux contraintes mécaniques subies lors de son utilisation, peu coûteux et léger en comparaison des corps d'impacteur actuels.

Le corps d'impacteur selon l'invention peut comprendre une armature rigide, p.ex. métallique, qui est recouverte au moins partiellement d'une enveloppe, p.ex. en polymère.

Par «armature rigide » on entend une structure offrant suffisamment de résistance mécanique et de résistance aux chocs subis lors de son utilisation. De préférence l'armature est en métal et l'enveloppe en polymère.

De façon avantageuse, l'armature rigide se présente sous la forme d'une ou plusieurs pièces, p.ex. en acier inoxydable, de géométrie simple permettant de les produire à moindre coût. De préférence, l'armature est ensuite placée dans un moule d'injection dans lequel l'enveloppe en polymère est injectée. L'enveloppe est donc surmoulée sur l'armature.

L'armature métallique offre au corps de l'impacteur la résistance mécanique et la résistance aux chocs nécessaire à son utilisation. L'enveloppe en polymère surmoulée permet de maintenir l'armature métallique en place, en évitant par exemple tout effet de flambage, et lui permettant donc de travailler selon un mode de déformation avantageux. Elle permet également de monter les autres pièces constituant l'impacteur. Elle a en outre pour avantage de rendre l'outil ergonomique, facilement nettoyable et élégant tout en diminuant fortement sa masse et son coût de fabrication par rapport à un corps réalisé entièrement en métal.

Selon un premier mode de réalisation, le polymère constituant l'enveloppe possède une bonne résistance à la stérilisation vapeur. Le corps d'impacteur ainsi obtenu est réutilisable.

Selon un second mode de réalisation, le polymère constituant l'enveloppe possède une faible résistance à la stérilisation vapeur. Le corps d'impacteur ainsi obtenu est à usage unique.

Selon un troisième mode de réalisation, le polymère utilisé est suffisamment solide et rigide pour se passer d'armature rigide. Dans ce cas, seule la zone de frappe peut être en métal, partiellement surmoulée par le polymère.

Un second objectif de l'invention consiste à guider la chaîne cinématique par la géométrie de l'enveloppe en polymères. Cette dernière possède une goulotte en « U » de la même largeur que la chaîne cinématique. Cette goulotte, combinée avec la matière dont est composée l'enveloppe, a un effet de palier et permet de guider la chaîne cinématique, avec un minimum de frottements et sans composants supplémentaires. La partie centrale du corps de l'impacteur, ainsi que la goulotte étant courbes, cela permet de venir appuyer la chaîne cinématique au fond de la goulotte, de manière tangente.

### Description sommaire des dessins

La présente invention et ses avantages apparaîtront mieux dans la description ci-dessous d'un mode de réalisation donné à titre d'exemple non limitatif, en référence aux dessins annexés dans lesquels :
- la figure 1 représente l'armature métallique du corps de l'impacteur avant le surmoulage de l'enveloppe,
- la figure 2 représente le corps d'impacteur après le surmoulage de l' enveloppe,
- la figure 3 représente une vue en coupe du corps de l'impacteur après le surmoulage de l'enveloppe. On distingue dans cette vue l'armature enclavée dans l'enveloppe.
- la figure 4 représente une vue en coupe du corps d'impacteur après surmoulage et avec uniquement une tête de frappe en métal.
- la figure 5 représente une vue en coupe du corps de l'impacteur, montrant la forme du logement de la chaîne cinématique.
- les figures 6 et 7 représentent l'impacteur avec une chaîne cinématique mise dans le corps d'impacteur et guidée par celui-ci. La chaîne vient s'appuyer, de manière tangente avec le fond du logement.

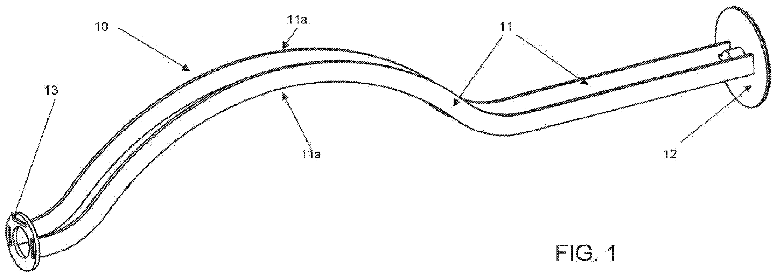

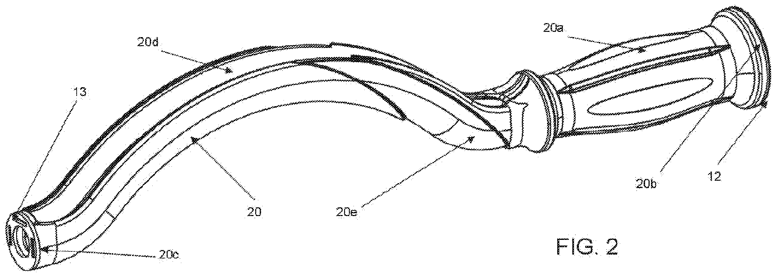

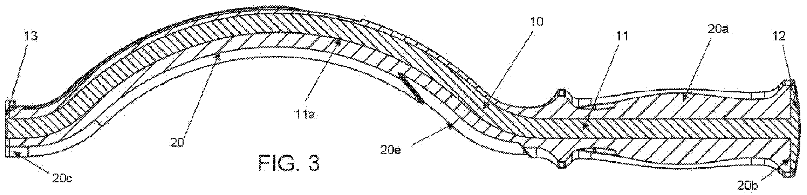

En référence aux figures 1 à 3, le corps d'impacteur comprend un élément allongé 20,11 a composé d'une armature en acier inoxydable 10,11,11 a et d'une enveloppe 20 en polymère 20. Une chaîne cinématique (30) est mise à l'intérieur du corps d'impacteur.

L'armature 10,11,11a est composée de plusieurs pièces simples en acier inoxydable, telles que les âmes 11, la tête de frappe 12 et la butée de cupule 13, maintenues ensembles afin de réaliser un sous-ensemble monobloc avant la phase d'injection.

L'enveloppe 20 est monobloc. Elle est de préférence en polymère et est injectée directement sur l'armature 10,11,11 a qui est préalablement placée dans le moule d'injection. L'enveloppe comprend également une poignée 20a, un appui de la tête de frappe 20b, un appui de la butée de cupule 20c et un logement 20d permettant le passage et le guidage d'une chaîne cinématique 30

La principale contrainte mécanique est appliquée lorsque le chirurgien frappe sur l'impacteur avec un marteau. Le marteau impacte sur la tête de frappe 12. La force est transmise de la tête de frappe 12 dans l'armature rigide 10,11,11 a puis dans la butée de cupule 13, 20c et finalement dans la cupule (non illustrée). La force se propage donc toujours au travers de pièces métalliques pouvant la supporter. L'armature rigide comprend en outre des âmes 11a en forme d'arc de cercle et de section constante, répartissant les contraintes de façon homogène dans les âmes, évitant donc tout pic de contrainte et donc toute détérioration locale.

Le procédé de surmoulage a pour effet de faire adhérer l'enveloppe 20 sur toutes les parois de l'armature rigide 10,11,11 a. Cette adhésion renforce la rigidité du corps de l'impacteur. Elle permet également de supprimer des degrés de liberté à l'armature rigide, faisant ainsi travailler mécaniquement celles-ci en compression et en flexion dans sa dimension la plus importante, limitant donc ses contraintes internes.

La poignée 20a de l'enveloppe 20 permet une prise en main ergonomique de l'impacteur. Le logement des pièces de l'impacteur 20d permet le montage et la mise en place de la chaîne cinématique 30. L'enveloppe 20 est réalisée de façon à être facilement nettoyable et à éviter toute détérioration des équipements médicaux utilisés par le chirurgien, notamment ses gants.

Selon une variante, le polymère constituant l'enveloppe 20 possède une bonne résistance à la stérilisation vapeur. Le corps d'impacteur ainsi obtenu est réutilisable.

Selon une autre variante, le polymère constituant l'enveloppe 20 possède une faible résistance à la stérilisation vapeur. Le corps d'impacteur ainsi obtenu est à usage unique.

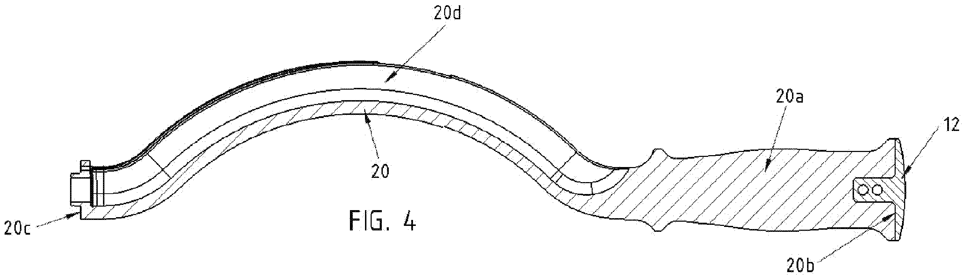

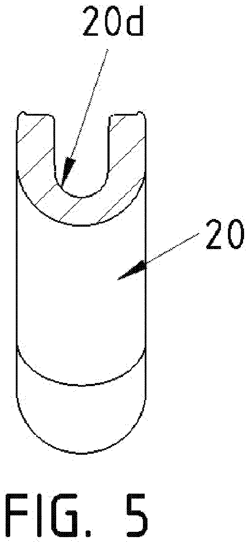

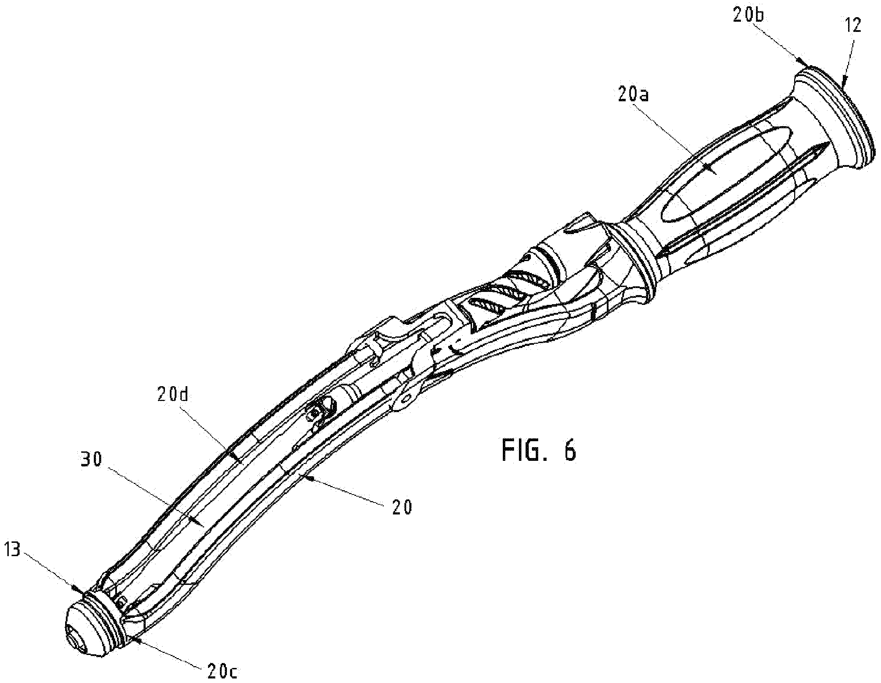

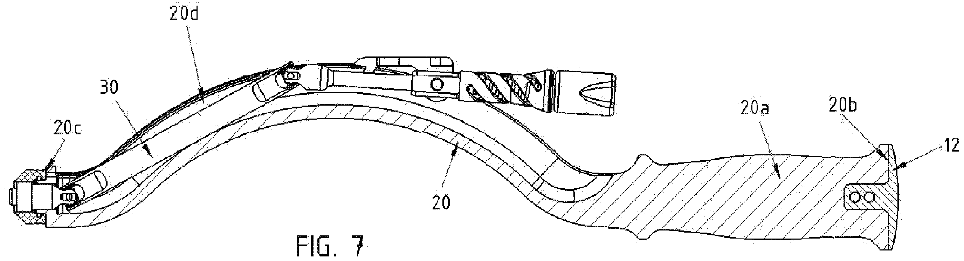

Selon un autre mode de réalisation (voir figures 4 à 7), le polymère constituant l'enveloppe 20 est suffisamment solide et rigide pour que le corps d'impacteur soit dépourvu d'une armature rigide. Dans ce cas, on conserve toutefois de préférence une zone de frappe 12 constituée d'un autre matériau, plus rigide, p.ex. en métal. L'extrémité distale 20c de l'enveloppe forme la butée de la cupule.

Le principe de l'invention n'est pas limité aux formes de réalisation décrites, mais est susceptible d'être modifié dans le cadre des revendications annexées.

## Revendications

1. Corps d'impacteur pour opération de chirurgie orthopédique comprenant une tête de frappe (12), une poignée (20a), une butée de cupule (13,20c) et un élément allongé (20) à l'intérieur duquel est disposé un conduit (20d) adapté pour loger une chaîne cinématique (30), **caractérisé par le fait que** ledit élément allongé (20) est au moins partiellement recouvert d'une enveloppe (20).

2. Corps d'impacteur selon la revendication 1 dans lequel l'élément allongé (20) comprend une armature rigide (10,11,11a), qui est au moins partiellement recouverte par ladite enveloppe (20).

3. Corps d'impacteur selon la revendication 2 dans lequel l'enveloppe (20) est surmoulée sur l'armature rigide (10,11,11a).

4. Corps d'impacteur selon l'une quelconque des revendications précédentes dans lequel l'enveloppe (20) est réalisée avec un polymère possédant une bonne résistance à la stérilisation vapeur, rendant le corps d'impacteur réutilisable.

5. Corps d'impacteur selon l'une quelconque des revendications précédentes 1 à 3 dans lequel l'enveloppe (20) est réalisée avec un polymère possédant une faible résistance à la stérilisation vapeur, rendant le corps d'impacteur à usage unique.

6. Corps d'impacteur selon l'une quelconque des revendications 2 à 5 dans lequel l'armature rigide comprend des âmes (11a) en forme d'arc de cercle et de section constante visant à répartir les contraintes liées à la frappe de façon uniforme dans celles-ci.

7. Corps d'impacteur selon l'une quelconque des revendications précédentes, dans lequel l'enveloppe (20) est rigide.

8. Corps d'impacteur selon la revendication 4 dépourvu d'armature rigide.

9. Corps d'impacteur selon la revendication 8 dans lequel la poignée (20a) est constituée par une extension de l'enveloppe (20).

10. Corps d'impacteur selon la revendication 9 dans lequel la tête de frappe (12) est constituée d'un matériau distinct, p.ex. en métal, de celui qui forme la poignée (20a).

11. Procédé de fabrication d'un corps d'impacteur tel que défini dans l'une quelconque des revendications 1 à 7comprenant les étapes suivantes :
- disposition de l'armature rigide (10,11,11a) dans un moule d'injection,
- injection de polymère dans ledit moule,
- formation et disposition de ladite enveloppe (20) par surmoulage de l'armature rigide (10,11,11a).

## Patentansprüche

1. Impaktor körper für die orthopädische Chirurgie umfassend einen Schlagkopf (12), eine Griff (20a), einen Becheranschlag (13, 20c) und ein langgestrecktes Element (20), in dessen Innerem eine Röhre (20d) angeordnet ist, die zur Aufnahme einer kinematischen Kette (30) geeignet ist, **dadurch gekennzeichnet, dass** das langgestreckte Element (20) zumindest teilweise von einem Mantel (20) umhüllt ist.

2. Impaktor körper nach Anspruch 1, wobei das langgestreckte Element (20) eine Versteifung (10, 11, 11a) umfasst, die zumindest teilweise von dem Mantel (20) umhüllt ist.

3. Impaktor körper nach Anspruch 2, wobei der Mantel (20) auf die Versteifung (10, 11, 11a) aufgegossen ist.

4. Impaktor körper nach einem der vorhergehenden Ansprüche, wobei der Mantel (20) aus einem Polymer mit guter Beständigkeit gegen Dampfsterilisation hergestellt ist, wodurch der Impaktor körper wiederverwendbar wird.

5. Impaktor körper nach einem der vorhergehenden Ansprüche 1 bis 3, wobei der Mantel (20) aus einem Polymer mit geringer Beständigkeit gegen Dampfsterilisation hergestellt ist, wodurch der Impaktor körper zum Einwegartikel wird.

6. Impaktor körper nach einem der Ansprüche 2 bis 5, wobei die Versteifung kreisbigenförmige Stege (11a) von konstantem Querschnitt umfasst, um die mit dem Aufprall verbundenen Spannungen gleichmäßig darin zu verteilen.

7. Impaktor körper nach einem der vorhergehenden Ansprüche, wobei der Mantel (20) starr ist.

8. Impaktor körper nach Anspruch 4 ohne Versteifung.

9. Impaktor körper nach Anspruch 8, wobei der Griff (20a) durch eine Verlängerung des Mantels (20) ausgebildet ist.

10. Impaktor körper nach Anspruch 9, wobei der Schlagkopf (12) aus einem Material besteht, das sich von dem, das den Griff (20a) bildet, unterscheidet, z. B. Metall.

11. Verfahren zur Herstellung eines Impaktor körpers nach einem der Ansprüche 1 bis 7, umfasst die folgenden Schritte:
- Anorden der Versteifung (10, 11, 11a) in einer Spritzgussform,
- Einspritzen von Polymer in die Form,
- Bilden und Anordnen des Mantels (20) durch Umspritzen der Versteifung (10, 11, 11 a).

## Claims

1. Impactor body for orthopaedic surgery operation, comprising a strike head (12), a handle (20a), a cup abutment (13, 20c), and an elongate element (20) inside which there is a conduit (20d) designed to house a kinematic chain (30), **characterized in that** said elongate element (20) is at least partially covered by an envelope (20).

2. Impactor body according to Claim 1, in which the elongate element (20) comprises a rigid frame (10, 11, 11a), which is at least partially covered by said envelope (20).

3. Impactor body according to Claim 2, in which the envelope (20) is overmoulded on the rigid frame (10, 11, 11a).

4. Impactor body according to any one of the preceding claims, in which the envelope (20) is made from a polymer having good resistance to steam sterilization, making the impactor body reusable.

5. Impactor body according to any one of Claims 1 to 3, in which the envelope (20) is made from a polymer having low resistance to steam sterilization, making the impactor body usable just once.

6. Impactor body according to any one of Claims 2 to 5, in which the rigid frame comprises webs (11a) in the shape of an arc of a circle and of constant cross section, which are provided to ensure that the stresses associated with the strike are distributed uniformly within them.

7. Impactor body according to any one of the preceding claims, in which the envelope (20) is rigid.

8. Impactor body according to Claim 4, having no rigid frame.

9. Impactor body according to Claim 8, in which the handle (20a) is formed by an extension of the envelope (20).

10. Impactor body according to Claim 9, in which the strike head (12) is made of a material, e.g. metal, distinct from that of the handle (20a).

11. Method for producing an impactor body as defined in any one of Claims 1 to 7, comprising the following steps:
- placing the rigid frame (10, 11, 11a) in an injection mould,
- injecting polymer into said mould,
- forming and arranging said envelope (20) by overmoulding of the rigid frame (10, 11, 11a).
